# EUROPÄISCHE PATENTSCHRIFT

(11) **EP 1 173 094 B1**
(45) Veröffentlichungstag und Bekanntmachung des Hinweises auf die Patenterteilung: **13.02.2008**
(21) Anmeldenummer: 00918566.1
(22) Anmeldetag: 19.04.2000
(51) Int. Cl.: A61B 5/0408

(54) **Verfahren zum Herstellen einer Neutralelektrode**
Method for manufacturing a neutral electrode
Procédé de fabrication d'une électrode neutre

(30) Priorität: 29.04.1999 AT 77099
(43) Veröffentlichungstag der Anmeldung: 23.01.2002
(73) Patentinhaber: Leonhard Lang KG, 6010 Innsbruck (AT)
(72) Erfinder: LANG, Burrhus, A-6020 Innsbruck (AT)
(74) Vertreter: Torggler, Paul Norbert
(86) Internationale Anmeldenummer: PCT/AT2000/000097
(87) Internationale Veröffentlichungsnummer: WO 2000/065992

(56) Entgegenhaltungen:
- EP-A- 0 509 710
- WO-A-93/00857
- WO-A-96/32057
- WO-A-97/24156
- US-A- 5 520 683

## Beschreibung

Die Erfindung betrifft ein Verfahren zum Herstellen von medizinischen Elektroden, insbesondere Neutralelektroden, bei dem ein bahnförmiges Laminat aus einem nichtleitenden Zwischenträger und einer Leiterschicht auf der Seite der Leiterschicht mit einem elektrisch leitenden, klebenden Gel beschichtet wird und die Laminat-Gel-Kombination konturgeformt - vorzugsweise ausgestanzt - wird, bevor sie auf der dem Gel abgewandten Seite auf einem Träger befestigt wird.

Medizinische Elektroden werden zu den verschiedensten Zwecken auf die Haut des Patienten geklebt, um elektrische Signale aufzunehmen bzw. Ströme ein- und abzuleiten (Defibrillationsneutralelektroden). Vor allem bei den medizinischen Bioelektroden, die als Neutralelektroden zum Einsatz kommen, um Ströme abzuleiten, ist es wichtig, daß der Hautkontakt möglichst gleichmäßig erfolgt, um lokale hohe Stromdichten zu vermeiden. Es sind bereits mehrschichtige Neutralelektroden bekannt, die auf der der Haut abgewandten Seite einen Träger beispielsweise aus Schaumstoff aufweisen. Als nächste Schicht folgt ein Laminat aus einem Zwischenträger mit einer Leiterschicht, beispielsweise Aluminium. Diese Leiterschicht ist dann hautseitig mit einem elektrisch leitenden, klebenden Gel (beispielsweise ein hautfreundliches Hydrogel) abgedeckt. Dabei ist darauf zu achten, daß die metallische Leiterschicht keinen direkten Kontakt zur Haut des Patienten bekommt.

Es ist bekannt, den Rand der Leiterschicht mit Gel oder einem zusätzlichen Isolationselement abzudecken. Diese Konstruktion ist jedoch aufwendig und teuer.

Um ein abdeckendes Isolationselement auszuformen, muß Material von annähernd derselben Fläche wie die der gesamten Elektrode eingesetzt werden, wobei der überwiegende Anteil Verschnitt und somit Abfall ist.

Wird die Leiterfläche mit Gel überlappend abgedeckt, so geschieht das nach einem von zwei bekannten Verfahren.

Zum einen kann eine flächige Gelbahn erzeugt, in der für die Überlappung nötigen Form ausgestanzt und präzise über die Leiterfläche geklebt werden. Dieses Verfahren ist sehr materialaufwendig, da zusätzliche Abdeckbahnen im Prozeßverlauf eingesetzt werden müssen, und stellt hohe Anforderungen an die Manipulation der klebrigen Gelfläche.

Zum anderen kann eine flüssige Vormischung für das Gel in ein dafür vorgesehenes Reservoir gegossen werden, das vom Träger der Elektrode samt Leitfläche (bzw. einer Abdekkung) und einem umlaufenden Randelement gebildet wird. Diese Vormischung polimerisiert dann, beispielsweise unter Zuführung von Energie, zu einem klebrigen Hydrogel. Auch hier fällt zur Ausbildung des umlaufenden Randelementes erheblicher Abfall an und ist das Handling der flüssigen Vormischung äußerst anspruchsvoll und fehleranfällig. Aufgrund dessen werden derartige Verfahren nur von wenigen (möglicherweise nur einem einzigen) Hersteller eingesetzt.

Aufgabe der Erfindung ist es, ein Verfahren anzugeben, mit dem sich eine medizinische Elektrode in einem automatisierten Herstellungsprozeß herstellen läßt, bei der es zu einer gleichmäßigen und sicheren Stromübertragung zwischen der Haut des Patienten und der Elektrode kommt.

Das erfindungsgemäße Verfahren ist in Anspruch 1 definiert.

Das erfindungsgemäße Verfahren ist dadurch gekennzeichnet, daß die Leiterschicht bereits bei der Herstellung des Laminates auf dem bahnförmigen Zwischenträger durch Konturformung - vorzugsweise durch vorhergehendes Ausstanzen - als nicht den gesamten bahnförmigen Zwischenträger bedeckende Felder aufgebracht wird, und daß die - vorzugsweise durch Ausstanzen erfolgte - Konturformung der Laminat-Gel-Kombination derart erfolgt, daß das Gel die Außenkanten der im Laminat vorgeformten Leiterschicht-Felder zumindest bereichsweise seitlich abdeckt.

Die Grundidee des erfindungsgemäßen Verfahrens besteht zunächst darin, für die Herstellung der Elektrode nicht wie üblich ein bahnförmiges Laminat zu verwenden, bei dem auf einem bahnförmigen, nichtleitenden Zwischenträger einfach eine gleich große bahnförmige Leiterschicht auflaminiert ist, sondern bereits bei der Herstellung ein spezielles Laminat vorzusehen, bei dem auf dem nichtleitenden Zwischenträger im wesentlichen diskrete Einzelfelder angeordnet werden. Dies erlaubt es, nach dem Aufbringen des leitfähigen Gels in einem weiteren Stanzprozeß eine Stanzung vorzunehmen, die im wesentlichen allseitig einen seitlichen Abstand zu den elektrisch leitenden Einzelfeldern aufweist. Im Gegensatz zum Stand der Technik, bei dem man das Gel samt der Leiterschicht gemeinsam gestanzt hat, erreicht man nunmehr mit dem erfindungsgemäßen Verfahren, daß das Gel die Außenkanten der im Laminat vorgeformten Leiterschicht-Felder seitlich abdeckt. Man kann damit eine Elektrode herstellen, bei der die leitfähige, klebende Gelschicht die leitfähige (Metall)Folie überall dort vollständig überlappt, wo ein Kontakt zum Körper besteht. Es werden damit hohe lokale Stromstärken durch direkten Kontakt des Leiters mit der Haut, die beispielsweise zu Verbrennungen führen können, vermieden. Die zum Anschluß an die Elektrodenkabel nötigen Lappen kann man selbstverständlich auch bei den Elektroden ohne Gelbedeckung frei abstehen lassen.

Das vorgeschlagene Verfahren ist viel einfacher im Prozeß als der Stand der Technik, da eine vollflächige Gelbeschichtung erfolgen kann. Es ist viel materialsparender, da keinerlei zusätzliche Materialien eingesetzt werden müssen (der Zwischenträger wird standardmäßig für Aluminiumlaminate verwendet (zur Aluminium-Ersparnis, gegen Knittern, für bessere Zugfestigkeit)).

Weitere Vorteile und Einzelheiten der Erfindung werden anhand der nachfolgenden Figuren-beschreibung näher erläutert.
Die Fig. 1 zeigt eine Einrichtung zur Herstellung eines bahnförmigen Laminates für die Durchführung eines Ausführungsbeispieles des erfindungsgemäßen Verfahrens.
Die Figuren 2b, 2c und 2d zeigen anhand der verwendeten Komponenten die Herstellung des erfindungsgemäßen Laminates.
Die Fig. 3 zeigt ein Ausführungsbeispiel einer Einrichtung zum Herstellen von medizinischen Elektroden, welcher ein bahnförmiges Laminat zugeführt wird, das beispielsweise wie in Fig. 1 hergestellt worden ist.
Die Figuren 3a, 3b und 3c zeigen verschiedene Herstellungstufen bei der erfindungsgemäßen Herstellung von Elektroden.
Die Fig. 4 zeigt Elektroden in einem Querschnitt kurz vor dem letzten Schneide- bzw. Stanzschritt zum Vereinzeln der Elektroden.

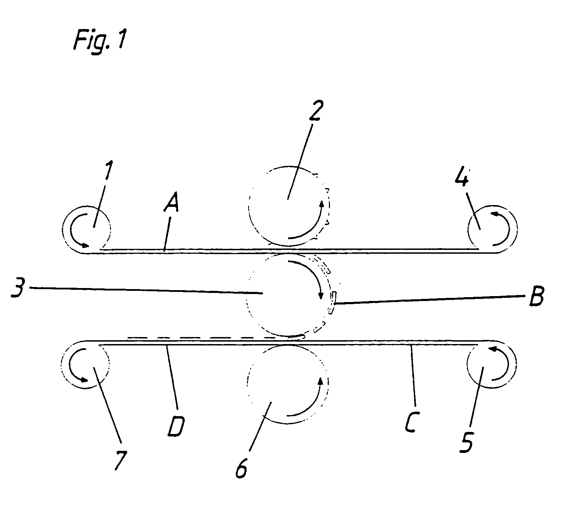

Unter Bezugnahme auf Fig. 1 wird von einer Rolle 1 eine bahnförmige Leiterschicht A, beispielsweise eine hochleitfähige Folie aus Aluminium mit einer Stärke von 25 Mikrometern abgerollt und durch eine Rotationsstanze 2 gefördert. In der Rotationsstanze 2 entstehen Stanzlinge, die in ihrer Endform bereits der Form der elektrisch leitenden Schicht in der später hergestellten fertigen Elektrode entsprechen. Es ist allerdings möglich, daß diese Stanzlinge B aus herstellungstechnischen Gründen durch dünne Stege miteinander verbunden sind. Die Stanzlinge B werden von einer Gegendruckwalze 3 weiter gefördert, das nicht mehr zu nutzende Stanzgitter wird auf der Rolle 4 aufgewickelt.

Von der Rolle 5 wird ein bahnförmiger, nichtleitender Zwischenträger C in Form einer PET-Folie von 40 Mikrometern Stärke abgerollt und zu einer Laminierwalze 6 gefördert. Zwischen der Laminierwalze 6 und der Gegendruckwalze 5 werden die hochleitfähigen Stanzlinge B mit dem nichtleitenden Zwischenträger C verbunden. Dies kann zum Beispiel dadurch erfolgen, daß die leitfähige Aluminiumfolie B an ihrer, der nicht leitfähigen PET-Folie C zugewandten Seite, mit einem thermisch aktivierbaren Klebstoff beschichtet ist, welcher mittels einer in der Laminierwalze 6 untergebrachten Heizvorrichtung aktiviert wird. Das bahnförmige Laminat D mit dem nichtleitenden Zwischenträger C und den darauf auflaminierten elektrisch leitenden Feldern B der Leiterschicht wird zur weiteren Verarbeitung weiter gefördert, beispielsweise auf die Rolle 7 aufgerollt.

Wesentlich ist die Tatsache, daß die Leiterschicht nicht als durchgehende Bahn auf dem nichtleitenden, bahnförmigen Zwischenträger C auflaminiert wird, sondern nur in "Einzelfeldern", wobei diese Einzelfelder vorzugsweise bereits der Endform der leitfähigen Schicht in den fertigen Elektroden entsprechen.

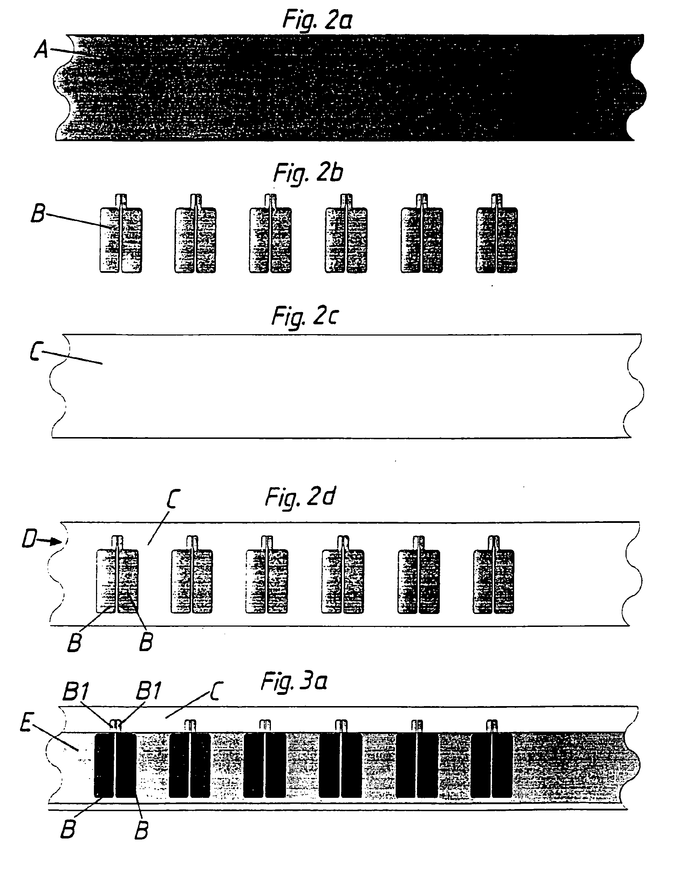

Die Fig. 2a zeigt die noch bahnförmige Leiterschicht (beispielsweise eine Aluminiumfolie A). Aus dieser Leiterschicht werden durch Konturformung, beispielsweise Stanzen, Stanzlinge bzw. allgemein Felder erzeugt, die dann auf die in Fig. 2c gezeigte nichtleitende Zwischenträgerbahn C aufgebracht werden. Schließlich erhält man als Ergebnis dieses ersten Arbeitsschrittes gemäß Fig. 2d ein Laminat D mit einem bahnförmigen, nichtleitenden Zwischenträger C und darauf aufgebrachten leitenden Feldern B.

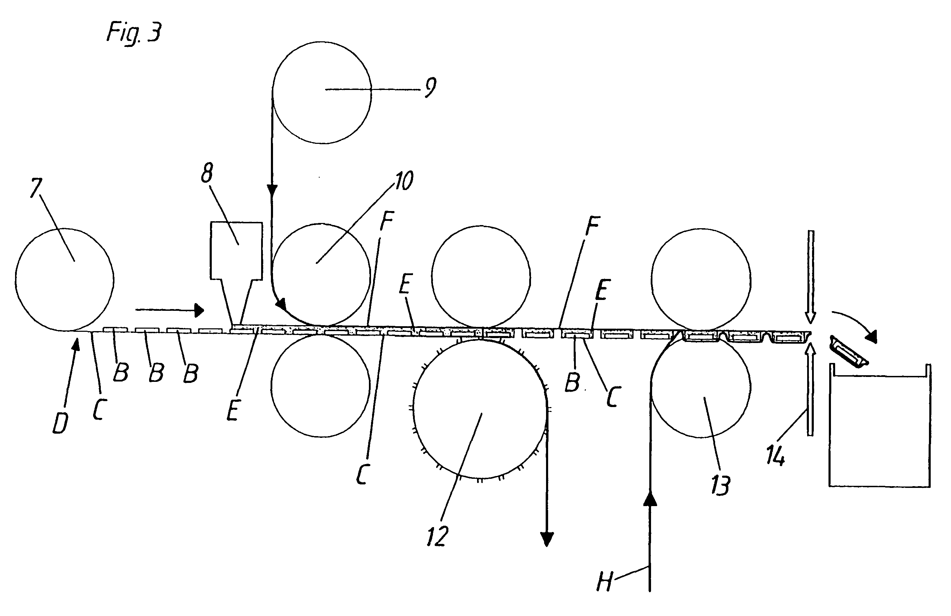

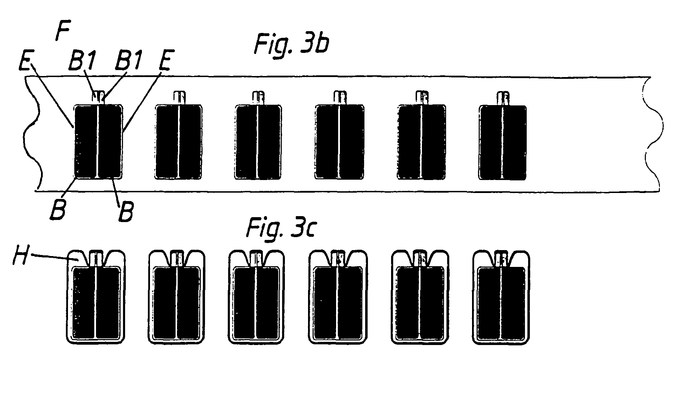

Dieses bahnförmige Laminat wird nun weiter verarbeitet, um die medizinischen Elektroden herzustellen. Dies kann beispielsweise in einer Einrichtung erfolgen, wie sie in Fig. 3 gezeigt ist. Von einer Rolle 7 wird das Laminat abgerollt. Es weist den bahnförmigen, nichtleitenden Zwischenträger C mit den darauf befindlichen elektrisch leitenden Feldern B auf. Dieses Laminat wird in der Station 8 mit einem klebrigen, leitfähigen Gel E durchgehend beschichtet, wobei die späteren Anschlußlaschen B1 frei bleiben können, wie dies die Fig. 3a zeigt. Auf diese Gelschicht kommt dann von einer Vorratsrolle über eine Andrückrolle 10 eine Abdeckschicht F aus abhäsivem Material (zum Beispiel einer silikonisierten Kunststoffolie).

In der Rotationsstanze 12 wird nun der Zwischenträger und die Gelschicht E, nicht aber die Abdeckschicht F, durchstanzt, und zwar so, daß das Gel E seitlich gegenüber den Außenkanten der im Laminat D vorgeformten Leiterschicht-Felder B zumindest bereichsweise (bis auf die Anschlußlaschen B1) übersteht. Man hat dann die in Fig. 3b gezeigte Situation. Wichtig ist, daß die Außenkanten bzw. Ränder der Leiterschicht-Felder B, die später über das Gel E mit der Haut in Kontakt treten, sicher abgedeckt sind und damit nicht direkt mit der Haut in Kontakt treten können. Auf die in Fig. 3b gezeigte Bahn wird nun in der Station 13 eine Trägerschicht aus einseitig klebrigem Material auflaminiert und anschließend in der Vereinzelungsstation (Schneiden bzw. Stanzen) die fertigen Einzelelektroden hergestellt, wie sie schematisch in der Fig. 3c gezeigt sind. Es wird noch erwähnt, daß die in Fig. 3 gezeigten Leiterschicht-Felder der Einfachheit halber durchgehend gezeichnet sind, während sie gemäß den Figuren 3a bis 3c pro Elektrode jeweils zweifach ausgeführt sind.

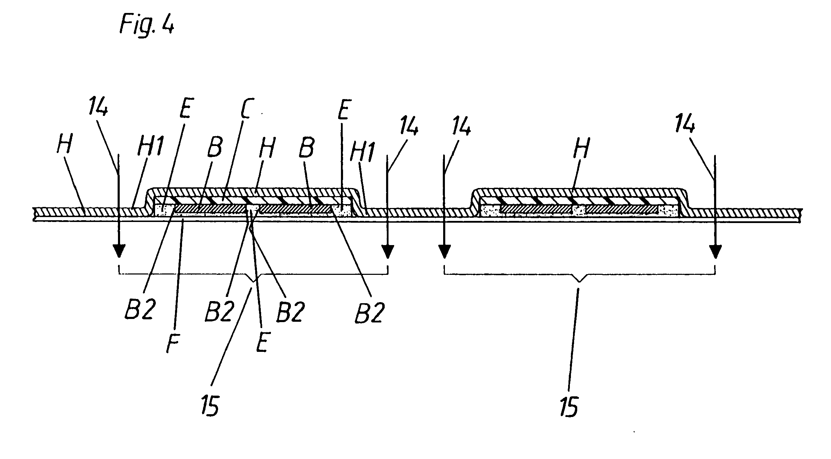

Die genauere Struktur einer solchen medizinischen Elektrode ist in Fig. 4 dargestellt. Jede Elektrode 15 wird über eine Vereinzelungsoperation 14, beispielsweise Schneiden oder Stanzen, von der im automatisierten Verfahrensprozeß verwendeten Bahn hergestellt. Der Schichtaufbau ist dabei der folgende: auf der der Haut abgewandten Seite befindet sich ein Trägermaterial H aus Schaumstoff, das auf der Unterseite klebrig ist. Dieses Schaumstoffmaterial weist seitlich überstehende Lappen H1 auf, die direkt auf der Haut des Patienten verklebt werden können. Unter diesem Stoffmaterial befindet sich ein Laminat, bestehend aus einem Zwischenträger C und elektrisch leitfähigen Feldern B. Gegenüber der Haut des Patienten sind diese leitfähigen Schichten B durch ein elektrisch leitfähiges, klebriges Gel E seitlich abgedeckt, wobei das Gel E bis zum Zwischenträger C reicht.

In den Zeichnungen sind die Dicken der Schichten aus darstellungstechnischen Gründen wesentlich dicker gezeichnet, als dies in der Praxis der Fall ist. Tatsächlich ist es günstig, wenn die Dicke der Leiterschicht B in der Größenordnung von 20 bis 15 Mikrometern und die Dicke des Zwischenträgers im Bereich von 20 bis 60 Mikrometern liegt. Außerdem sind in den Zeichnungen die Klebeschichten auf der der Haut zugewandten Seite des Trägers H und zwischen den leitfähigen Feldern B und dem Zwischenträger C nicht als gesonderte Schichten dargestellt.

Zusammenfassend läßt sich feststellen, daß das Ausführungsbeispiel des erfindungsgemäßen Verfahrens insgesamt drei Stanzschritte umfaßt, nämlich einen ersten Stanzschritt bei der Herstellung des bahnförmigen Laminates gemäß Fig. 1 zum bereichsweisen Aufbringen der Leiterschicht auf den Zwischenträger. Ein zweiter Stanzschritt stanzt das leitfähige Gel samt dem Zwischenträger in einem gewissen Abstand um die vorgeformten Leiterschicht-Felder herum. In einem dritten Stanzschritt wird dann der als Träger fungierende Schaumstoff samt der silikonisierten Abdeckschicht durchstanzt, um die einzelnen Elektroden zu formen.

## Patentansprüche

1. Verfahren zum Herstellen von medizinischen Elektroden, insbesondere Neutralelektroden, bei dem ein bahnförmiges Laminat aus einem nichtleitenden Zwischenträger und einer Leiterschicht auf der Seite der Leiterschicht mit einem elektrisch leitenden, klebenden Gel beschichtet wird und die Laminat-Gel-Kombination konturgeformt - vorzugsweise ausgestanzt - wird, bevor sie auf der dem Gel abgewandten Seite auf einem Träger befestigt wird, wobei die Leiterschicht (A) bereits bei der Herstellung des Laminates (D) auf dem bahnförmigen Zwischenträger (C) als nicht den gesamten bahnförmigen Zwischenträger (C) bedeckende Felder (B) aufgebracht wird, wobei die Felder (B) aus einer Leiterschicht-Bahn (A) durch Konturformung - vorzugsweise Stanzen - gebildet werden, und wobei die - vorzugsweise durch Ausstanzen erfolgte - Konturformung der Laminat-Gel-Kombination (D,E) derart erfolgt, daß das Gel (E) die Außenkanten der im Laminat (D) vorgeformten Leiterschicht-Felder (B) zumindest bereichsweise,vorzugsweise im wesentlichen allseitig, seitlich abdeckt.

2. Verfahren nach Anspruch 1, **dadurch gekennzeichnet, daß** die Leiterschicht-Felder (B) auf den bahnförmigen Zwischenträger (C) aufgeklebt werden, vorzugsweise zwischen einer Laminierwalze (6) und einer Gegendruckwalze (5) unter Verwendung eines thermoaktivierbaren Klebers.

3. Verfahren nach Anspruch 1 oder 2, **dadurch gekennzeichnet, daß** die Leiterschicht-Felder (B) durch Ausstanzen aus der Leiterschicht-Bahn (A) unter Verwendung einer Rotationsstanze (2) gebildet werden.

4. Verfahren nach Anspruch 2 und 3, **dadurch gekennzeichnet, daß** die Rotationsstanze (2) mit einer Gegendruckwalze (5) zusammenarbeitet, welche die als Stanzlinge vorliegenden Leiterschicht-Felder (B) der Laminierwalze (6) zum Auflaminieren auf den bahnförmigen Zwischenträger (C) zuführt.

5. Verfahren nach einem der Ansprüche 1 bis 4, **dadurch gekennzeichnet, daß** die Form der Leiterschicht-Felder (B) im Laminat (D) bereits der Endform der Leiterschicht in den fertigen Elektroden (15) entspricht.

6. Verfahren nach einem der Ansprüche 1 bis 5, **dadurch gekennzeichnet, daß** das bahnförmige Laminat (D) auf der Seite der Leifierschicht-Felder (B) mit einer vorzugsweise durchgehenden Bahn eines elektrisch leitenden, klebrigen Gels (E) beschichtet wird.

7. Verfahren nach einem der Ansprüche 1 bis 6, **dadurch gekennzeichnet, daß** das Gel (E) - vorzugsweise nach dem Aufbringen auf das Laminat (D) - mit einer bahnförmigen Abdeckschicht (F), vorzugsweise einer silikonisierten Kunststoffolie, abgedeckt wird.

8. Verfahren nach Anspruch 7, **dadurch gekennzeichnet, daß** die Konturformung der Laminat-Gel-Kombination (D, E) durch Ausstanzen auf der Abdeckschicht (F) erfolgt, wobei lediglich der Zwischenträger (C) des Laminats und das Gel (E), nicht aber die Abdeckschicht (F), durchstanzt werden und als Stanzlinge auf der Abdeckschicht (F) verbleiben.

9. Verfahren nach einem der Ansprüche 1 bis 8, **dadurch gekennzeichnet, daß** der Träger als Trägerbahn (H) auf der dem Gel (E) abgewandten Seite mit den konturgeformten Laminat-Gel-Feldern verbunden, vorzugsweise verklebt wird, wobei vorzugsweise die Trägerbahn (H) selbst einseitig klebrig ausgebildet ist.

10. Verfahren nach Anspruch 8 und 9, **dadurch gekennzeichnet, daß** die Trägerbahn (H) mit der mit den Stanzlingen versehenen Abdeckschicht (F) verbunden wird, woraus anschließend die fertigen Elektroden (15) vereinzelt, vorzugsweise ausgestanzt werden.

## Claims

1. A process for producing medical electrodes, in particular neutral electrodes, wherein a laminate in web form comprising a non-conductive intermediate carrier and a conductor layer is coated on the side of the conductor layer with an electrically conductive adhesive gel and the laminate-gel combination is shaped to a contour, preferably stamped out, before it is fixed on a carrier on the side remote from the gel, wherein the conductor layer (A) is already applied in manufacture of the laminate (D) to the intermediate carrier in web form (C) - preferably by preceding stamping-out - in the form of fields (B) which do not cover the entire intermediate carrier in web form (C), and wherein the operation of shaping the laminate-gel combination (D, E) to contour - which is preferably effected by stamping out - is effected in such a way that the gel (E) laterally projects at least in a region-wise manner, preferably substantially, at all sides with respect to the outer edges of the conductor layer fields (B) which are pre-formed in the laminate (D).

2. A process as set forth in claim 1 **characterized in that** the conductor layer (B) fields are adhesively fixed on the intermediate carrier in web form (C), preferably between a laminating roller (6) and a counter-pressure roller (5), using a heat-activatable adhesive.

3. A process as set forth in claim 1 or claim 2 **characterized in that** the conductor layer fields (B) are formed by stamping out of a conductor layer web (A), preferably using a rotary stamping apparatus (2).

4. A process as set forth in claim 2 and claim 3 **characterized in that** the rotary stamping apparatus (2) co-operates with a counter-pressure roller (5) which feeds the conductor layer fields (B) in the form of stampings to the laminating roller (6) for lamination onto the intermediate carrier in web form (C).

5. A process as set forth in one of claims 1 through 4 **characterized in that** the form of the conductor layer fields (B) in the laminate (D) already corresponds to the final form of the conductor layer in the finished electrodes (15).

6. A process as set forth in one of claims 1 through 5 **characterized in that** the laminate in web form (D) is coated on the side of the conductor layer fields (B) with a preferably continuous web of an electrically conductive, adhesive gel (E).

7. A process as set forth in one of claims 1 through 6 **characterized in that** the gel (E) is covered - preferably after application to the laminate (D) - with a cover layer in web form (F), preferably a siliconized plastic foil.

8. A process as set forth in claim 7 **characterized in that** contour shaping of the laminate-gel combination (D, E) is effected by stamping out on the cover layer (F), wherein only the intermediate carrier (C) of the laminate and the gel (E) but not the cover layer (F) are stamped through and remain as stampings on the cover layer (F).

9. A process as set forth in one of claims 1 through 8 **characterized in that** the carrier is connected, preferably by adhesive means, in the form of a carrier web (H), on the side remote from the gel (E), to the contour-shaped laminate-gel fields, wherein preferably the carrier web (H) itself is adhesive on one side.

10. A process as set forth in claim 8 and claim 9 **characterized in that** the carrier web (H) is joined to the cover layer (F) provided with the stampings, from which then the finished electrodes (15) are individually separated, preferably by being stamped out.

## Revendications

1. Procédé de fabrication d'électrodes médicales, en particulier d'électrodes neutres, dans lequel un stratifié en forme de bande, formé par un support intermédiaire non conducteur et une couche conductrice, est revêtu sur la face de la couche conductrice par un gel adhésif électroconducteur, et la combinaison formée par le stratifié et le gel est soumise à un formage des contours - de préférence, par découpe -, avant qu'elle ne soit fixée sur un substrat avec la face opposée au gel, la couche conductrice (1) étant déjà déposée, lors de la fabrication du stratifié (D), sur le support intermédiaire (C) en forme de bande sous forme de zones (B) ne recouvrant pas la totalité du support intermédiaire (C) en forme de bande, les zones (B) étant formées à partir d'une bande de couche conductrice (A) par formage des contours - de préférence, par découpe -, et le formage des contours - réalisé de préférence par découpe - de la combinaison formée par le stratifié et le gel (D, E) étant effectué de telle sorte que le gel (E) couvre latéralement au moins par zones, de préférence sensiblement sur tous les côtés, les bords extérieurs des zones de couche conductrice (B) préformées dans le stratifié (D).

2. Procédé selon la revendication 1, **caractérisé en ce que** les zones de couche conductrice (B) sont collées sur le support intermédiaire (C) en forme de bande, de préférence entre un cylindre de laminage (8) et un cylindre de contre-pression (5) moyennant l'utilisation d'une colle thermoactivable.

3. Procédé selon la revendication 1 ou 2, **caractérisé en ce que** les zones de couche conductrice (B) sont formées par découpe dans la bande de couche conductrice (A) moyennant l'utilisation d'un outil de découpe (2) rotatif.

4. Procédé selon les revendications 2 et 3, **caractérisé en ce que** l'outil de découpe (2) rotatif coopère avec un cylindre de contre-pression, par lequel les zones de couche conductrice (B) disponibles sous forme de découpes sont acheminées vers le cylindre de laminage (8) pour être déposées par laminage sur le support intermédiaire (C) en forme de bande.

5. Procédé selon l'une quelconque des revendications 1 à 4, **caractérisé en ce que** la forme des zones de couche conductrice (B) dans le stratifié (D) correspond déjà à la forme définitive de la couche conductrice dans les électrodes (15) finies.

6. Procédé selon l'une quelconque des revendications 1 à 5, **caractérisé en ce que** le stratifié (D) en forme de bande est revêtu, sur 1.a face des zones de couche conductrice (B), d'une bande de préférence continue d'un gel (E) adhésif électroconducteur.

7. Procédé selon l'une quelconque des revendications 1 à 6, **caractérisé en ce que** le gel (E) - de préférence après son application sur le stratifié (D) - est recouvert par une couche de protection (F) en forme de bande, de préférence un film plastique siliconé.

8. Procédé selon la revendication 7, **caractérisé en ce que** le formage des contours de la combinaison formée par le stratifié et le gel (D, E) est obtenu par découpe dans la couche de protection (F), sachant qu'uniquement le support intermédiaire (C) du stratifié et le gel (E), et non pas la couche de protection (F), étant poinçonnées et subsistent sous forme de découpes sur la couche de protection (F).

9. Procédé selon l'une quelconque des revendications 1 à 8, **caractérisé en ce que** le support sous forme de bande de support (H), sur la face opposée au gel (E), est assemblé, de préférence collé, avec les zones du stratifié et gel à contours formés, la bande de support (H) étant elle-même de préférence adhésive sur une face.

10. Procédé selon les revendications 8 et 9, **caractérisé en ce que** la bande de support (H) est assemblée à la couche de protection (F), munie des découpes, les électrodes (15) finies étant ensuite détaillées, de préférence découpées, dans cet assemblage obtenu.
